# EUROPEAN PATENT APPLICATION

(11) **EP 4 831 673 A1**
(43) Date of publication of application: **28.10.2026**
(21) Application number: 25172648.5
(22) Date of filing: 25.04.2025
(51) Int. Cl.: G01R 33/56, G01R 33/565, A61B 5/055

(54) **IMAGE RECONSTRUCTION IN MAGNETIC RESONANCE IMAGING DEPENDING ON DATA CONSISTENCY**

(71) Applicant: Siemens Healthineers AG, 91301 Forchheim (DE)
(72) Inventor: Cauley, Stephen Farman, Winchester, 01890 (US); Clifford, Bryan, Belmont, 02478 (US); Polak, Daniel, 91052 Erlangen (DE); Splitthoff, Daniel Nicolas, 91080 Uttenreuth (DE)
(74) Representative: Siemens Healthineers Patent Attorneys

(57) **Abstract**

For image reconstruction in magnetic resonance imaging, MRI, MRI data (4) representing an object is received. A first preliminary image (5a) is reconstructed based on the MRI data (4). A first data consistency measure (6a) of the first preliminary image (5a) with respect to the MRI data (4) is computed. A final image (8a, 8b) is reconstructed based on the MRI data (4), wherein, depending on the first data consistency measure (6a), a motion of the object during an acquisition of the MRI data (4) is either compensated in the final image (8a, 8b) based on predetermined motion data (7), which quantifies the motion of the object during the acquisition of the MRI data (4), or is not compensated in the final image (8a, 8b). Therein, a predefined image quality of the first preliminary image (5a) is less than the image quality of the final image (8a, 8b), wherein the image quality is an image size or a spatial resolution.

## Description

The present invention is directed to a computer-implemented method for image reconstruction, in particular retrospective image reconstruction, in magnetic resonance imaging, MRI. The invention is further directed to a data processing system for carrying out such a computer-implemented method, to an MRI system comprising such a data processing system and to a corresponding computer program product.

Patient motion during the acquisition of MRI data can lead to significant artifacts or distortions in the reconstructed images. Motion correction techniques to reduce artifacts in MRI images are known. For clinical applications, it is crucial that these methods work with high reliability, effectively mitigating artifacts when motion is present, while preserving image quality when the patient remains still. Achieving a high level of robustness is challenging due to technical limitations, including errors in motion tracking. These inaccuracies can sometimes lead to degradations in the image quality of the images. In some instances, images corrected for motion may even appear worse than uncorrected images, for example with subtle ringing or more pronounced motion artifacts emerging as a result of the correction techniques.

In general, motion correction techniques for MRI may be classified as prospective or retrospective motion correction techniques. In prospective motion correction, adjustments are made in real time to manage motion artifacts as they occur. This approach has a limited flexibility due to the need for immediate response but may include real-time filtering techniques such as the Kalman filter or threshold-based field-of-view updating.

Retrospective techniques allow for additional processing time, facilitating a comprehensive analysis of motion data and images. For example, motion trajectory filtering approaches involve smoothing or adjusting the estimated motion trajectory before applying any corrections.

In retrospective approaches using automated correction decision-making, an algorithm evaluates whether to display the corrected or uncorrected image to the user based on the quality. Both direct, image-based methods and indirect, trajectory-based methods may be used. For image-based quality assessment, techniques based on image entropy or using deep learning networks have been developed to automatically evaluate and select the highest-quality image. However, reliably fine-tuning of these metrics to consistently distinguish image quality can be challenging, especially across varying sequence protocols, contrast settings, and noise conditions. Additionally, these approaches require generating both the corrected and uncorrected images, which is computationally intensive. If the corrected image ultimately proves inferior, this process results in unnecessary reconstruction time. Alternatively, decisions can be made by analyzing the estimated motion trajectory. Metrics such as maximum translation and/or rotation, motion standard deviation, or other statistical measures can be applied. While these methods avoid the need for explicit image reconstruction, they are typically less reliable, often requiring a balance between sensitivity and specificity to optimize the results.

The publication D. Polak et al.: "Scout Accelerated Motion Estimation and Reduction (SAMER)", Magn. Reson. Med., 2022, 87(1), 163-178 describes a technique denoted as SAMER, which utilizes a single short, low-resolution scout scan and sequence reordering to independently determine motion states by minimizing the data-consistency error in a SENSE plus motion forward model.

The publication D. Polak et al.: "Motion guidance lines for robust data-consistency based retrospective motion correction in 2D and 3D MRI", Magn. Reson. Med., 2023, 89(5), 1777-1790 proposes a motion correction technique based on repeating k-space guidance lines for improving motion correction in cartesian 2D and 3D brain MRI.

The publication L. Cordero-Grande et al.: "Sensitivity Encoding for Aligned Multishot Magnetic Resonance Reconstruction", IEEE Transactions on Computational Imaging, 2016, vol. 2, no. 3 describes a framework for the reconstruction of magnetic resonance images in the presence of rigid motion.

It is an objective of the present invention to provide an improved concept for retrospective motion compensation, also denoted as motion correction, in MRI, which features low computational costs and a high reliability.

This objective is achieved by the subject matter of the independent claims. Further implementations and preferred embodiments are subject matter of the dependent claims.

The invention is based on the idea to use a data consistency of a reconstructed preliminary image, which has a reduced image quality, with respect to the underlying MRI data as a criterion for deciding whether the final image is reconstructed with or without motion compensation.

According to an aspect of the invention a computer-implemented method for image reconstruction in magnetic resonance imaging, MRI, is provided. Therein, MRI data, in particular measured MRI data, representing an object, in particular an imaged object, is received. A first preliminary image is reconstructed based on the MRI data. A first data consistency measure of the first preliminary image with respect to the MRI data is computed. A final image is reconstructed based on the MRI data, wherein a predefined image quality of the first preliminary image is less than the image quality of the final image. Furthermore, depending on the first data consistency measure, a motion of the object during an acquisition of the MRI data is either compensated in the final image, in particular when reconstructing the final image, based on predetermined motion data, which quantifies the motion of the object during the acquisition of the MRI data, or the motion of the object is not compensated in the final image.

Unless stated otherwise, all steps of the computer-implemented method may be performed by a data processing system, which comprises at least one data processing device. In particular, the at least one data processing device is configured or adapted to perform the steps of the computer-implemented method. For this purpose, the at least one data processing device may for example store a computer program comprising instructions which, when executed by the at least one data processing device, cause the at least one data processing device to execute the computer-implemented method. The expressions "data processing system" and "at least one data processing device" may be used interchangeably, here and in the following. This holds also for respective expressions derived therefrom.

In case the at least one data processing device comprises two or more data processing devices, certain steps carried out by the at least one data processing device may also be understood such that different data processing devices carry out different steps or different parts of a step. In particular, it is not required that each data processing device carries out the steps completely. In other words, carrying out the steps may be distributed amongst the two or more data processing devices.

From each implementation of the computer-implemented method, a respective implementation of a method for image reconstruction in MRI, which is not purely computer-implemented, is obtained by including respective steps of generating the MRI data, in particular by an MRI device.

Receiving data or information, in particular the MRI data, may, for example, involve receiving the data, in particular by the data processing system from a sending entity, or reading the data from a data storage or receiving a data stream containing the data, or extracting the data from the data stream, and so on. In particular, wired or wireless data transmission can be used for this purpose. In particular, the data transmission may take place between a hardware and/or software interface of the sending entity and a hardware and/or software interface of the data processing system.

A reconstructed image may also be denoted as MRI image. A reconstructed image may also be denoted as image reconstruction. A reconstructed image may be a two-dimensional or a three-dimensional image, the latter also being denoted as reconstructed volume. A reconstructed image may also be a two-dimensional slice or layer of a three-dimensional reconstructed image. In particular, the first preliminary image and the final image may also be denoted as first preliminary MRI image and final MRI image, respectively.

The MRI data are, in particular, MRI data in k-space or in a hybrid image-k-space. The MRI data may comprise different datasets, which correspond for example to different data acquisition shots of the acquisition of the MRI data and/or different k-space regions. The different datasets of the MRI data may also comprise datasets corresponding to preliminary scans or navigation scans or scout scans, such as the SAMER scout scan.

Consequently, it is possible that the MRI data are not used completely for reconstructing the final image and/or for reconstructing the first preliminary image and so forth. In particular, in some embodiments, only a part of the MRI data is used to reconstruct the first preliminary image. In this case, the first data consistency measure is computed for the first preliminary image with respect to the part of the MRI data, which has been used for reconstructing the first preliminary image.

Considering a reconstructed MRI image, it can be transferred back into k-space by applying the corresponding forward operator of the acquisition to the reconstructed MRI image. A data consistency measure may for example be a measure, which quantifies a deviation between the k-space data resulting from the back transferred MRI image and the original k-space data, which has been used to reconstruct the MRI image. The data consistency measure may therefore be of the form of a data consistency error $\left‖E x-y\right‖ ,$ wherein *x* denotes the reconstructed MRI image *E* denotes a predefined forward operator and y denotes the original k-space data. The forward operator may, for example, comprise a Fourier transform, respective coil sensitivity maps in case of parallel imaging and a projection to the sampled k-space points in case of k-space subsampling. The forward operator may also comprise or depend on the motion data, for example.

The predefined image quality may for example be an image size or a resolution, in particular a spatial resolution, or another image quality, whose reduction leads to a reduced computational effort for reconstructing the respective image. Consequently, the computational resources required for reconstructing the first preliminary image are less than the computational resources required for reconstructing the final image in case all other relevant parameters would be identical.

According to the invention, it is decided depending on the first data consistency measure whether the motion of the object is compensated using the motion data when reconstructing the final image or not. In particular, the motion of the object is compensated when reconstructing the final image only if it is determined based on the first data consistency measure that a sufficient benefit can be expected due to the motion compensation. In other words, motion compensation is avoided if the benefit is expected to be low or if the motion compensation could even lead to a worse result than achievable without motion compensation. Thus, the computational effort is, on average, significantly reduced without risking significant artifacts due to object motion during the acquisition.

Another advantage is that explicit image-based metrics, such as image entropy, which are often challenging to tune and may not consistently yield reliable results, are not required for the computer-implemented method according to the invention.

For reconstructing the first preliminary image and final image depending on the MRI data, known methods for MRI reconstruction may be used. In particular, if motion compensation depending on the motion data shall be included, known techniques for retrospective motion correction may be used.

Also the motion data may be generated using known techniques including, for example, navigator scans, scout scans, camera-based motion tracking, optimization methods and so forth.

In particular, the motion data may comprise or consists of one, two, or three translation parameters as a function of time during the acquisition of the MRI data and/or one, two, or three rotation parameters as a function of time during the acquisition of the MRI data. In other words, the motion of the object is assumed to be a rigid motion. Alternatively, whole motion vector fields may be used, which also allows for considering non-rigid motion.

According to several embodiments, the image quality is a spatial resolution. Consequently, the spatial resolution of the first preliminary image is less than the spatial resolution of the final image.

In other words, the number of k-space samples used or considered to reconstruct the first preliminary image is less than the number of k-space samples used or considered to reconstruct the final image.

Reducing the number of k-space samples for reconstructing the first preliminary image compared to reconstructing the first preliminary image significantly reduces the computational effort for reconstructing the first preliminary image. On the other hand, object motion typically affects certain regions of the k-space, for example regions close to the k-space center, much more than other regions, in particular region further away from the k-space center. Thus, by selecting the k-space samples used for reconstructing the first preliminary image accordingly, the influence of the motion of the object on the first preliminary image and, consequently, the first data consistency measure, is not significantly different compared to a case where all k-space samples would be used. The first preliminary image is therefore still a suitable measure for deciding whether the final image should be reconstructed with or without motion compensation.

According to several embodiments, the motion of the object during the acquisition of the MRI data is compensated in the first preliminary image, in particular when reconstructing the first preliminary image, based on the motion data.

In this way it is achieved that the influence of the motion of the object on the first preliminary image is reduced or completely removed. The first data consistency measure can therefore serve as a reference measure for what can be expected from the motion compensation for the final image.

According to several embodiments, a second preliminary image is reconstructed based on the MRI data, wherein the motion of the object is not compensated in the second preliminary image, in particular when reconstructing the second preliminary image, and the image quality of the second preliminary image is less than the image quality of the final image. A second data consistency measure of the second preliminary image with respect to the MRI data is computed. A data consistency deviation between the first data consistency measure and the second data consistency measure is computed. The final image is reconstructed based on the MRI data, wherein, depending on the data consistency deviation, the motion of the object is either compensated based on the motion data in the final image or is not compensated in the final image.

According to such embodiments, it is decided depending on the data consistency deviation whether the motion of the object is compensated using the motion data when reconstructing the final image or not.

For example, the data consistency deviation may be a difference or an absolute value of the difference between the first data consistency measure and the second data consistency measure.

For example, the image quality of the second preliminary image may be equal to the image quality of the first preliminary image. For example, the first preliminary image and the second preliminary image may be reconstructed based on the same part of the MRI data.

The second data consistency measure can serve as a reference measure for what can be expected for the final image if no motion compensation is carried out. The data consistency deviation may therefore be considered as a measure of the expected benefit of the motion compensation when reconstructing the final image.

For example, it may be provided that the motion of the object is compensated based on the motion data in the final image if the data consistency deviation is greater than a predefined threshold value and the motion of the object is not compensated in the final image otherwise.

According to several embodiments, the first data consistency measure depends on or is given by $\left‖{E}_{θ} x-y\right‖ ,$ wherein *x* denotes the first preliminary image, *θ* denotes the motion data, *E_{θ}* denotes a predefined forward operator, which depends on the motion data, and y denotes the part of the MRI data, which is used for generating the first preliminary image.

The motion data may be incorporated into the forward operator, for example, as described in the above-mentioned publication of L. Cordero-Grande et al. Furthermore, the forward operator may, for example, comprise a Fourier transform, respective coil sensitivity maps in case of parallel imaging and a projection to the sampled k-space points in case of k-space subsampling.

It is noted that data consistency measure as above are commonly used for iteratively reconstructing MRI images, for example minimizing the data consistency measure under variation of the image space data. Here, however, the data consistency measure is computed based on the already completely reconstructed first preliminary image and used to decide whether the final image shall be reconstructed with or without motion compensation.

For example, the second data consistency measure depends on or is given by $\left‖E x′-y′\right‖ ,$ wherein *x*' denotes the second preliminary image, *E* denotes the forward operator without the dependency on the motion data, and *y'* denotes the part of the MRI data, which is used for reconstructing the second preliminary image, wherein it may be provided that *y'* = *y.*

According to several embodiments, for reconstructing the first preliminary image, parts of the MRI data are weighted differently depending on respective sampled k-space locations.

For example, k-space regions may be weighted higher the closer they are located to the k-space center. Low-frequency components in k-space generally have higher signal-to-noise ratio, SNR, than peripheral high-frequency components. By assigning greater weight to the k-space center, which is where the most critical image information is located, the reliability of the method is improved leading to more accurate motion correction decisions.

According to several embodiments, for computing the first data consistency measure, parts of the MRI data are weighted differently depending on respective sampled k-space locations.

The first data consistency measure is then for example given by or depends on $\left‖w\left({E}_{θ} x-y\right)\right‖ ,$ wherein w denotes a respective weighting mask.

According to several embodiments, only a part of the MRI data for which the motion of the object is neglectable is used for reconstructing the first preliminary image. For example, the MRI data may comprise a dataset, which may also be denoted as preliminary scan dataset, which corresponds to a preliminary scan of the object, also denoted as scout scan, and the first preliminary image is reconstructed based on the preliminary scan dataset.

This may, in particular, be provided as an alternative for compensating the motion of the object when reconstructing the first preliminary image. For example, a scan duration of the scan generating the part of the MRI data which is used for reconstructing the first preliminary image may be very short such that the motion of the object is neglectable. Alternatively or in addition, if the object is a patient, the patient may be instructed not to move at all while the part of the MRI data which is used for reconstructing the first preliminary image is acquired.

The preliminary scan may for example be a SAMER scout scan as described in the 2022 publication of D. Polak et al. mentioned above.

For example, the MRI data comprises the preliminary scan dataset, the first preliminary image is reconstructed based on the preliminary scan dataset, and a duration of the preliminary scan is less than ten seconds or less than eight seconds or less than six seconds or less than four seconds. For example, the duration of the preliminary scan may lie in the range [0.5 s, 10 s] or in the range [0.5 s, 8 s] or in the range [0.5 s, 6 s] or in the range [0.5 s, 4 s].

In this way, it is achieved that the motion of the object is neglectable in the first preliminary image.

According to several embodiments, the MRI data comprises one or more first datasets corresponding to one or more first data acquisition shots of the acquisition of the MRI data and one or more second datasets corresponding to one or more second data acquisition shots of the acquisition of the MRI data. The one or more second data acquisition shots are carried out after the one or more first data acquisition shots. The first data consistency measure is computed based on the first preliminary image with respect to the one or more first datasets, in particular with respect to only the one or more first datasets. A second data consistency measure is computed based on the first preliminary image with respect to the one or more second datasets, in particular with respect to only the one or more second datasets. The final image is reconstructed based on the MRI data, wherein, depending on the first data consistency measure and the second data consistency measure, the motion of the object is either compensated in the final image based on the motion data or is not compensated in the final image.

In particular, the preliminary scan may be carried out prior to the one or more first data acquisition shots.

It is noted that each of the one or more first datasets does not necessarily contain all data acquired in the respective first data acquisition shot. In other words, the one or more first datasets correspond to the one or more first data acquisition shots but, in some embodiments, may contain only a part of the data acquired during the one or more first data acquisition shots, respectively.

According to such embodiments, it is decided depending on the first data consistency measure and the second data consistency measure whether the motion of the object is compensated using the motion data when reconstructing the final image or not. In particular, the motion of the object is compensated when reconstructing the final image only if it is determined based on the first data consistency measure and the second data consistency measure that a sufficient benefit can be expected due to the motion compensation.

It is noted that in previously described embodiments the first data consistency measure and the second data consistency measure are computed based on the motion-compensated and non-motion-compensated, respectively, in order to assess the benefit of a potential motion compensation of the final image. In the presently described embodiments, on the other hand, the same image, namely the preliminary first image, may for example be used to compute the two different data consistency measures with respect to data acquired at different times in order to assess the benefit of a potential motion compensation of the final image. This is based on the insight that a change in data consistency also may serve as a suitable indicator for said benefit of the motion compensation.

For example, the first data consistency measure depends on or is given by $\left‖{E}_{1} x-{y}_{1}\right‖ ,$ wherein *x* denotes the first preliminary image, *E*₁ denotes a predefined first forward operator, and *y*₁ denotes the one or more first datasets. In particular, *E*₁ depends on further first motion data, which quantifies the motion of the object during the one or more first data acquisition shots.

For example, the second data consistency measure depends on or is given by $\left‖{E}_{2} x-{y}_{2}\right‖ ,$ wherein *x* denotes the first preliminary image, *E*₂ denotes a predefined second forward operator, and *y*₂ denotes the one or more second datasets. In particular, *E*₂ depends on further second motion data, which quantifies the motion of the object during the one or more second data acquisition shots. In other words, the second data consistency measure is computed with motion compensation

The further data consistency measures may be computed analogously.

In some embodiments, the second data consistency measure is computed with motion compensation or, in other words, depending on the respective further second motion data.

In some embodiments, a reference second data consistency measure is computed based on the first preliminary image with respect to the one or more second datasets without compensating for object motion or, in other words, independent of the respective further second motion data or without taking into account the further second motion data. The final image is reconstructed based on the MRI data, wherein, depending on the first data consistency measure, the second data consistency measure and the reference second data consistency measure, the motion of the object is either compensated in the final image based on the motion data or is not compensated in the final image. For example, the final image is reconstructed based on the MRI data, wherein, depending on the first data consistency measure and depending on a difference between the second data consistency measure and the reference second data consistency measure, the motion of the object is either compensated in the final image based on the motion data or is not compensated in the final image.

In particular, the second data consistency measure and the reference second data consistency measure may be computed identically except that the respective further second motion is used for computing the second data consistency measure but is not used, for example by setting its values to zero, for computing the reference second data consistency measure.

In some embodiments, it may be provided that the first image is used to compute the two different data consistency measures for detecting parts of the data that should be excluded from an image reconstruction. Such data gaps may be compensated for by the rest of the data, in particular if the gaps are not too big.

According to several embodiments, a data consistency variation measure is computed depending on the first data consistency measure and the second data consistency measure. The final image is reconstructed based on the MRI data, wherein, depending on the data consistency variation measure, the motion of the object is either compensated in the final image based on the motion data or is not compensated in the final image.

In this case, it is decided depending on the data consistency variation measure whether the motion of the object is compensated using the motion data when reconstructing the final image or not. In particular, the motion of the object is compensated when reconstructing the final image only if it is determined based on the data consistency variation measure that a sufficient benefit can be expected due to the motion compensation.

For example, it may be provided that the motion of the object is compensated based on the motion data in the final image if the data consistency variation measure is greater than a predefined further threshold value and the motion of the object is not compensated in the final image otherwise.

The data consistency variation measure may for example be or depend on a difference between the first data consistency measure and the second data consistency measure or an absolute value of said difference.

It is, however, also possible that, apart from the one or more first data acquisition shots and the one or more second data acquisition shots, further data acquisition shots or sequences of further data acquisition shots are carried out and a respective further data consistency measure is computed for each further data acquisition shot or sequence of further data acquisition shots at different times. In this case the data consistency variation measure may be computed based on the first data consistency measure, the second data consistency measure, and the further data consistency measures. In this way, a more reliable estimation of the expected benefit of the motion compensation is possible.

In such embodiments, the data consistency variation measure may for example be or depend on a maximum difference of all pairs of the first data consistency measure, the second data consistency measure, and the further data consistency measures, a variance or standard deviation of the first data consistency measure, the second data consistency measure, and the further data consistency measures, et cetera.

Optionally, a respective reference data consistency measure may be computed for each of the further data acquisition shots without motion compensation as described above for the second data consistency measure. For computing the data consistency variation measure, instead of using the second data consistency measure and the further data consistency measures, the difference between the second data consistency measure and the reference second data consistency measure as well as the respective differences between the further data consistency measures and their respective reference further data consistency measures may be used.

The motion data may be determined in a known way as described above. The motion data may also be determined in an iterative approach or optimization approach as described in the 2022 publication of D. Polak et al. mentioned above. Therein, the motion data for a given acquisition shot is varied in multiple optimization steps in order to minimize a respective data consistency error, which is given by the difference between a term given by the preliminary scan dataset to which a forward operator is applied, which depends on the current motion data, from motion guidance data acquired during the respective acquisition shot.

It is noted that each of the one or more first datasets does not necessarily contain all data acquired in the respective first data acquisition shot. In particular, the one or more first datasets may correspond to the motion guidance data of the one or more first data acquisition shots and the one or more second datasets may correspond to the motion guidance data of the one or more second data acquisition shots. In other words, the procedure of determining the motion data is exploited additionally to track the data consistency in order to estimate the expected benefit of the motion-compensation in the reconstruction of the final image.

According to several embodiments, the data consistency variation measure takes into account the change in data consistency, in particular data consistency with motion compensation versus data consistency without motion compensation, over time.

According to several embodiments, a significance class is determined by applying a machine learning model, MLM, in particular a trained MLM, to input data, which depends on or comprises the first data consistency measure and the second data consistency measure and, for example, the further data consistency measures. The final image is reconstructed based on the MRI data, wherein, depending on the significance class, the motion of the object is either compensated in the final image based on the motion data or is not compensated in the final image.

The MLM is or has been, respectively, trained as a classification model to assign one of two or more significance classes to said data consistency measures. For example, a first data significance class of the two or more significance classes indicates a significant variation of the data consistency and a second significance class of the two or more significance classes indicates an insignificant variation of the data consistency. For example, it may be provided that the motion of the object is compensated based on the motion data in the final image if the first significance class is determined depending on said input data and the motion of the object is not compensated in the final image if the second significance class is determined depending on said input data.

In this way, a particularly reliable prediction of the estimation of the expected benefit of the motion-compensation in the reconstruction of the final image is possible.

In general terms, an MLM, in particular a trained MLM, may mimic cognitive functions that humans associate with other human minds. In particular, by training based on training data the MLM may be able to adapt to new circumstances and to detect and extrapolate patterns. Another term for a trained MLM is "trained function".

In general, parameters of an MLM can be adapted or updated by means of training. In particular, supervised training, semi-supervised training, unsupervised training, reinforcement learning and/or active learning can be used. Furthermore, representation learning, also denoted as feature learning, can be used. In particular, the parameters of the MLMs can be adapted iteratively by several steps of training. In particular, within the training a certain loss function, also denoted as cost function, can be minimized. In particular, within the training of an artificial neural network, ANN, the backpropagation algorithm can be used.

For example, an MLM can comprise an ANN, a support vector machine, a decision tree and/or a Bayesian network, and/or the MLM can be based on k-means clustering, Q-learning, genetic algorithms and/or association rules. In particular, an ANN can be or comprise a fully connected network, a multi-layer perceptron, MLP, a deep neural network, a convolutional neural network or a convolutional deep neural network. Furthermore, an ANN can be an adversarial network, a deep adversarial network and/or a generative adversarial network, GAN.

In some implementations, the MLM is trained in a supervised fashion based on a plurality of training datasets and ground truth data, each training dataset comprising a respective first data consistency measure, a respective second data consistency measure and, for example, respective further data consistency measures. The ground truth data includes respective annotations or ground truths for each training dataset, the ground truth comprising a respective ground truth significance class.

The MLM is applied to one of the training datasets to predict a respective predicted significance class. A predefined loss function is evaluated depending on the predicted significance class and the respective ground truth significance class, in particular depending on respective deviations of the predicted significance class from the respective ground truth significance class. The MLM is the adapted or updated depending on a result of the evaluation of the loss function. This is repeated for all training datasets or at least for a subset of the plurality of training datasets until a predefined termination criterion or convergence criterion is met.

According to several embodiments, for each dataset of a plurality of datasets of the MRI data, which correspond to a plurality of subsequent data acquisition shots of the acquisition of the MRI data, a respective data consistency measure is computed based on the first preliminary image with respect to the respective dataset. The final image is reconstructed based on the MRI data, wherein, depending on the data consistency measures computed for the plurality of datasets, the motion of the object is either compensated in the final image based on the motion data or is not compensated in the final image.

In particular, the plurality of subsequent data acquisition shots comprises the one or more first data acquisition shots, the one or more second data acquisition shots, and the further data acquisition shots. Consequently, the data consistency measures computed for the plurality of datasets comprise the first data consistency measure, the second data consistency measure, and the further data consistency measures.

In particular, a data consistency variation measure is computed, as described above, depending on the data consistency measures of the plurality of datasets. The final image is reconstructed based on the MRI data, wherein, depending on the data consistency measures computed for the plurality of datasets, the motion of the object is either compensated in the final image based on the motion data or is not compensated in the final image.

According to several embodiments, parts of the MRI data may be excluded from the process of reconstructing the final image based on the MRI data depending on the data consistency measures computed for the plurality of datasets.

For example, if it is found that certain data acquisition shots are affected particularly strongly by motion according to the data consistency measures, the respective k-space date may be ignored or not used, respectively, for reconstructing the final image. Thus, motion artifacts can be suppressed even more effectively.

According to several embodiments, significance class of the motion of the object is determined by classifying the motion of the object according to at least two significance classes based on the data consistency measures computed for the plurality of datasets. The final image is reconstructed based on the MRI data, wherein, depending on the determined significance class of the motion of the object, the motion of the object is either compensated in the final image based on the motion data or is not compensated in the final image.

The classification may be carried out by an MLM as described above or a by a conventional classification algorithm.

According to a further aspect of the invention, a data processing system that is configured to carry out a computer-implemented method according to the invention is provided.

In the present disclosure, the terms "data processing system" and "at least one data processing device" can be used interchangeably. In particular, a data processing device can be understood to mean a data processing device that contains a processing circuit. The data processing device can therefore, in particular, process data for the purpose of performing computing operations. This may also include operations for performing indexed access to a data structure, for example a look-up table, LUT, as well as a data processing method implemented in hardware.

The data processing device may include, in particular, one or more computers, one or more microcontrollers and/or one or more integrated circuits, for example one or more application-specific integrated circuits, ASIC, one or more field-programmable gate arrays, FPGA, and/or one or more systems-on-a-chip, SoC. The data processing device may also include one or more processors, for example one or more microprocessors, one or more central processing units, CPUs, one or more graphics processing units, GPUs, and/or one or more signal processors, in particular one or more digital signal processors, DSPs. The data processing device may also include a physical or virtual network of computers or other of the mentioned units.

In various embodiments, the data processing device includes one or more hardware and/or software interfaces and/or one or more storage units.

A storage unit may be a volatile data memory, for example a dynamic random access memory, DRAM, or a static random access memory, SRAM, or a non-volatile data memory, for example a read-only memory, ROM, a programmable read-only memory, PROM, an erasable programmable read-only memory, EPROM, an electrically erasable programmable read-only memory, EEPROM, a flash memory or flash EEPROM, a ferroelectric random access memory, FRAM, a magnetoresistive random access memory, MRAM, or a phase-change random access memory, PCRAM.

According to a further aspect of the invention, an MRI system is provided. The MRI system comprises a data processing system according to the invention and an MRI device, also denoted as MRI scanner. The MRI device is configured to generate the MRI data.

Further implementations of the MRI system according to the invention follow directly from the various embodiments of the method according to the invention and vice versa. In particular, individual features and corresponding explanations as well as advantages relating to the various implementations of the method according to the invention can be transferred analogously to corresponding implementations of the MRI system according to the invention. In particular, the MRI system according to the invention is designed or programmed to carry out the method for image reconstruction according to the invention. In particular, the MRI system according to the invention carries out the method for image reconstruction according to the invention.

According to a further aspect of the invention, a computer program product comprising instructions is provided. When the instructions are executed by a data processing system, the instructions cause the data processing system to carry out a computer-implemented method according to the invention.

The instructions may be provided as program code, for example. The program code can for example be provided as binary code or assembler and/or as source code of a programming language, for example C, and/or as program script, for example Python.

The computer program product may, for example, be a computer program with the instructions or a computer-readable storage medium, in particular a tangible and/or non-transient computer readable storage medium, storing a computer program with the instructions.

Further features and feature combinations of the invention are obtained from the figures and their description as well as the claims. In particular, further implementations of the invention may not necessarily contain all features of one of the claims. Further implementations of the invention may comprise features or combinations of features, which are not recited in the claims.

In the following, the invention will be explained in detail with reference to specific exemplary implementations and respective schematic drawings. In the drawings, identical or functionally identical elements may be denoted by the same reference signs. The description of identical or functionally identical elements is not necessarily repeated with respect to different figures.

In the figures,
- FIG 1: shows schematically an exemplary embodiment of an MRI system according to the invention;
- FIG 2: shows a schematic flow diagram of an exemplary embodiment of a computer-implemented method for image reconstruction in MRI according to the invention;
- FIG 3: shows a schematic flow diagram of a further exemplary embodiment of a computer-implemented method for image reconstruction in MRI according to the invention;
- FIG 4: shows a schematic flow diagram of a further exemplary embodiment of a computer-implemented method for image reconstruction in MRI according to the invention;
- FIG 5: shows a course of data consistency measures during an iterative MRI reconstruction; and
- FIG 6: shows a course of data consistency measures during a further iterative MRI reconstruction.

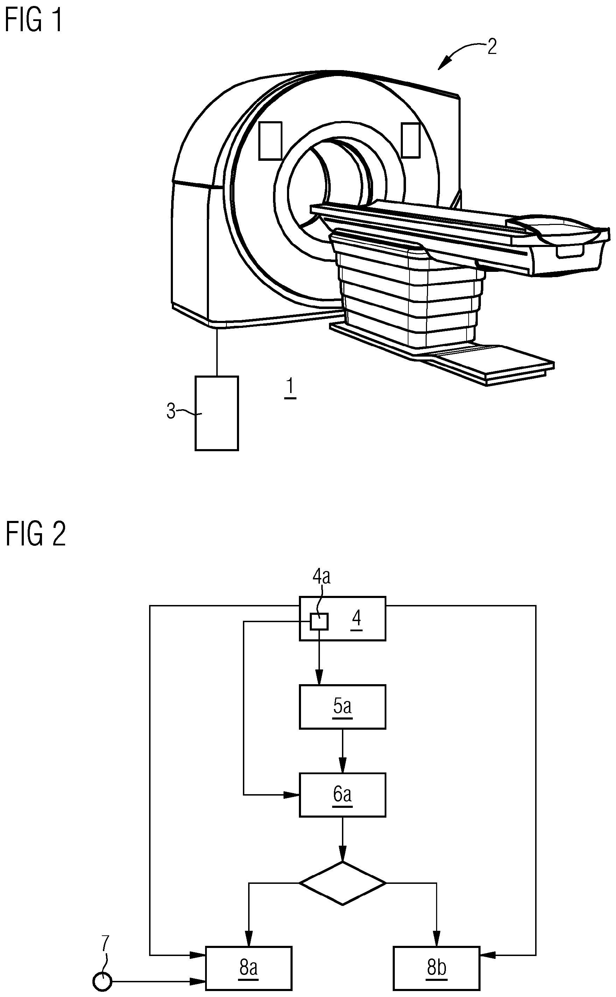

FIG 1 shows schematically an exemplary embodiment of an MRI system 1 according to the invention. The MRI system 1 comprises a data processing system 3 and an MRI device 2, which is configured to generate MRI data 4 (see FIG 2 to FIG 4) representing an object. The data processing system 3 is configured to carry out a computer-implemented method for image reconstruction in MRI based on the MRI data 4.

FIG 2 shows a schematic flow diagram of an exemplary embodiment of a computer-implemented method for image reconstruction in MRI according to the invention, which may be carried out by the data processing system 3 of FIG 1.

Therein, a first preliminary image 5a is reconstructed based on the MRI data 4, in particular based on a part 4a of the MRI data 4. A first data consistency measure 6a of the first preliminary image 5a with respect to the part 4a of the MRI data 4 is computed. A final image 8a, 8b is reconstructed based on the MRI data 4, in particular on the whole MRI data 4 or base on the part 4a and a further part of the MRI data 4. Therein, it is decided depending on the first data consistency measure 6a, whether or not a motion of the object during an acquisition of the MRI data 4 is compensated in the final image 8a, 8b based on predetermined motion data 7, which quantifies the motion of the object during the acquisition of the MRI data 4. If the motion is compensated, a motion-compensated final image 8a results and otherwise a non-motion-compensated final image 8b. In both cases, a predefined image quality, for example a spatial resolution, of the first preliminary image 5a is less than the image quality, for example the spatial resolution, of the final image 8a, 8b.

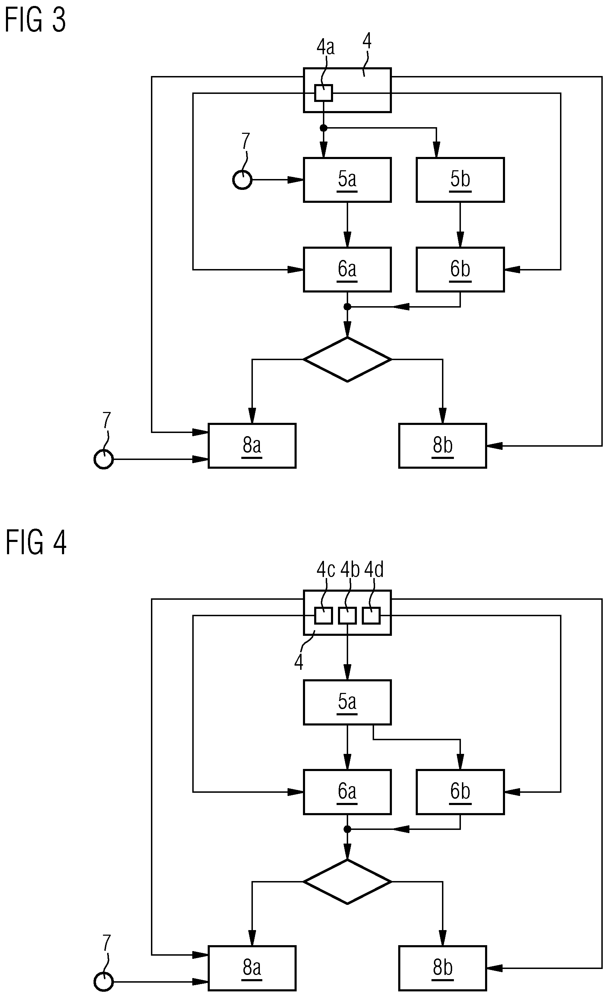

FIG 3 shows a schematic flow diagram of a further exemplary embodiment of a computer-implemented method for image reconstruction in MRI according to the invention, which is based on the method of FIG 2.

In the embodiment of FIG 3, a second preliminary image 5b is reconstructed based on the MRI data 4, in particular based on the part 4a of the MRI data 4. The image quality of the second preliminary image 5b is less than the image quality of the final image 8a, 8b. Therein, the motion of the object is compensated in the first preliminary image 5a based on the motion data 7 and is not compensated in the second preliminary image 5b.

A second data consistency measure 6b of the second preliminary image 5b with respect to the MRI data 4, in particular with respect to the part 4a, is computed.

For example, the first data consistency measure 6a may be given by $\left‖{E}_{θ} {x}_{1}-y\right‖ ,$ wherein *x*₁ denotes the first preliminary image 5a, *θ* denotes the motion data 7, *E_{θ}* denotes a predefined forward operator, which depends on the motion data 7, and y denotes the part 4a of the MRI data 4, which is used for generating the first preliminary image 5a. In particular, the motion data 7 comprises three translation parameters and three rotation parameters as a function of time during the acquisition of the MRI data 4.

Analogously, the second data consistency measure 6b may be given by $\left‖{E}_{0} {x}_{2}-y\right‖ ,$ wherein *x*₂ denotes the second preliminary image 5b, *E*₀ is equal to the forward operator *E_{θ}* except that no motion of the object is considered, or, in other words, the translation parameters and the rotation parameters are set to zero in *E*₀.

A data consistency deviation between the first data consistency measure 6a and the second data consistency measure 6b is computed, in particular as a difference between the first data consistency measure 6a and the second data consistency measure 6b. The final image 8a, 8b with or without the motion compensation depending on the data consistency deviation. For example, the motion-compensated final image 8a is reconstructed if the data consistency deviation is greater than a predefined threshold value and the non-motion-compensated final image 8b is reconstructed otherwise.

Embodiments according to FIG 3 may, for example, monitor the deviation of the so-called "SENSE+" data consistency errors (see the publication of L. Cordero-Grande et al. mentioned above) between the motion-corrected and uncorrected preliminary images 5a, 5b In general, the motion-corrected first preliminary image 5a is expected to align better with the acquired k-space data 4a than the uncorrected second preliminary image 5b, leading to a reduction in data consistency error in the latter. One significant advantage of this approach is that it eliminates the need for explicit image-based metrics, such as image entropy, which are often challenging to tune and may not consistently yield reliable results. This issue that reconstructions are rather time-consuming is mitigated by conducting the preliminary images 5a, 5b with reduced image quality. Based on the evaluation of the data consistency deviation, the algorithm can then trigger the more computationally intensive reconstruction of the high-resolution final image 8a, 8b and include motion correction only when necessary.

To further improve the reliability of the approach, non-rigid motion effects may be suppressed during reconstruction. In head imaging, for example, the brain can typically be modeled using rigid-body motion in very good approximation, while the neck or mouth region may exhibit non-rigid movement. Techniques such as readout cropping or channel mixing may be used to focus the reconstruction on the desired region, for example the brain, thereby effectively minimizing non-rigid motion effects and improving the accuracy of the data consistency evaluation.

Additionally, it may be beneficial to apply different weighting to regions of k-space. The low-frequency components of k-space generally have higher SNR than the peripheral high-frequency components. By assigning greater weight to the k-space center, where the most critical image information is located, the reliability of the proposed metric can be enhanced, leading to more accurate motion correction decisions.

FIG 4 shows a schematic flow diagram of a further exemplary embodiment of a computer-implemented method for image reconstruction in MRI according to the invention, which is based on the method of FIG 2.

In the embodiment of FIG 4, only a part of the MRI data 4 for which the motion of the object is neglectable is used for generating the first preliminary image. In particular, the MRI data 4 comprises a dataset 4b, which corresponds to a preliminary scan of the object, wherein a duration of the preliminary scan is less than ten seconds, for example in the range from two to six seconds, and the first preliminary image 5a is generated based on the dataset 4b.

Furthermore, the MRI data 4 comprises one or more first datasets 4c corresponding to one or more first data acquisition shots of the acquisition of the MRI data 4 and one or more second datasets 4d corresponding to one or more second data acquisition shots of the acquisition of the MRI data 4, wherein the one or more second data acquisition shots are carried out after the one or more first data acquisition shots. The first data consistency measure 6a is computed based on the first preliminary image 5a with respect to the one or more first datasets 4c and the second data consistency measure 6b is computed based on the first preliminary image 5a with respect to the one or more second datasets 4d. The final image 8a, 8b is reconstructed with or without the motion compensation based on the motion data 7 depending on the first data consistency measure 6a and the second data consistency measure 6b.

In particular, the approach of FIG 4 may be extended to more than just the two datasets 4c, 4d and the respective data consistency measures 6a, 6b. In particular, for each dataset of a plurality of datasets of the MRI data 4 including the datasets 4c, 4d, which correspond to a plurality of subsequent data acquisition shots including the first data acquisition shots and the second data acquisition shots, respectively, a respective data consistency measure 6a, 6b is computed based on the first preliminary image 5a with respect to the respective dataset 4c, 4d. The final image 8a, 8b is then reconstructed based on the MRI data 4 either with or without motion compensation depending on the data consistency measures 6a, 6b computed for the plurality of datasets 4c, 4d, in particular depending on a data consistency variation measure, which is determined based on the data consistency measures 6a, 6b. The data consistency variation measure may for example be a maximum difference of all pairs of the data consistency measures, or a variance or standard deviation of the data consistency measures.

In embodiments according to FIG 4, the motion correction decision may, in particular, be guided by tracking the per-shot data consistency errors from the motion estimation. This approach is based on the motion-free or approximately motion-free first preliminary image 5a as a reference, which may, for example, be obtained either after the final iteration of an alternating optimization method or directly from a SAMER scout scan. By passing the image estimate or scout through the motion forward model and comparing it against the acquired k-space data or motion guidance lines from each shot, changes in the data consistency over time can be monitored. For example, small, stable data consistency values suggest minimal or no motion, whereas larger fluctuations may indicate significant motion, prompting a motion-corrected reconstruction.

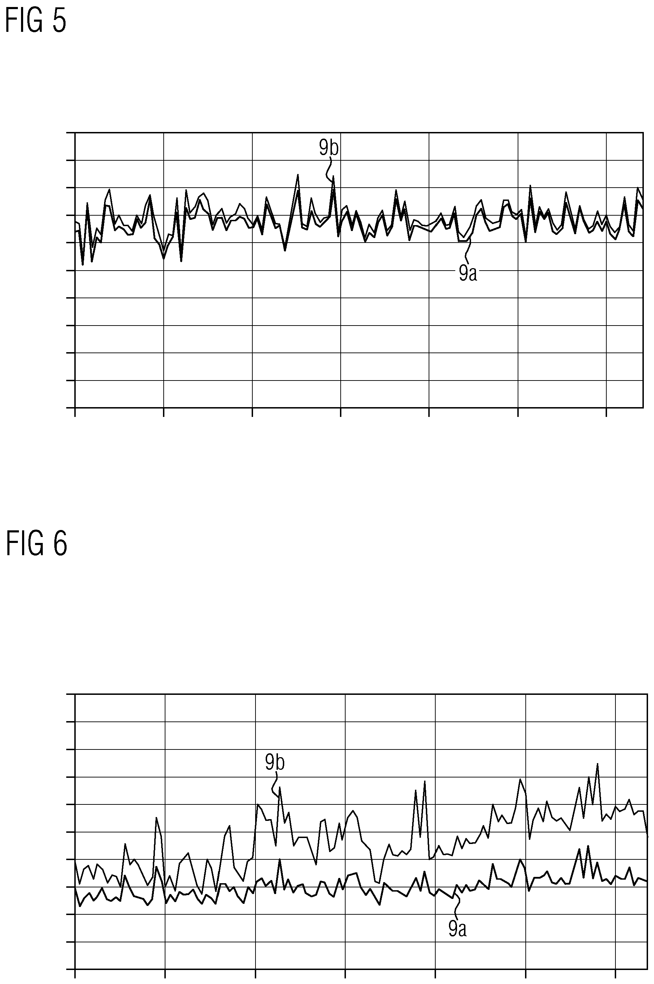

The data consistency measure is shown schematically in FIG 5 and FIG 6, wherein a first curve 9a represents the first data consistency measure 5a over many shots, while a second curve 9a represents the second data consistency measure 5b over many shots. In the case of FIG 5, the difference between the curves 9a, 9b is rather stable. Thus, the final image 8a, 8b could be reconstructed without motion compensation. In the case of FIG 6, the difference between the curves 9a, 9b increases strongly over time. Thus, the final image 8a, 8b could be reconstructed with motion compensation.

Independent of the grammatical term usage, individuals with male, female or other gender identities are included within the term.

## Claims

1. Computer-implemented method for image reconstruction in magnetic resonance imaging, MRI, wherein
- MRI data (4) representing an object is received;
- a first preliminary image (5a) is reconstructed based on the MRI data (4);
- a first data consistency measure (6a) of the first preliminary image (5a) with respect to the MRI data (4) is computed;
- a final image (8a, 8b) is reconstructed based on the MRI data (4), wherein, depending on the first data consistency measure (6a), a motion of the object during an acquisition of the MRI data (4) is either compensated in the final image (8a, 8b) based on predetermined motion data (7), which quantifies the motion of the object during the acquisition of the MRI data (4), or is not compensated in the final image (8a, 8b); and
- a predefined image quality of the first preliminary image (5a) is less than the image quality of the final image (8a, 8b).

2. Computer-implemented method according to claim 1, wherein the image quality is a spatial resolution.

3. Computer-implemented method according to one of the preceding claims, wherein the motion of the object during the acquisition of the MRI data (4) is compensated in the first preliminary image (5a) based on the motion data (7).

4. Computer-implemented method according to claim 3, wherein
- a second preliminary image (5b) is reconstructed based on the MRI data (4), wherein the motion of the object is not compensated in the second preliminary image (5b) and the image quality of the second preliminary image (5b) is less than the image quality of the final image (8a, 8b);
- a second data consistency measure (6b) of the second preliminary image (5b) with respect to the MRI data (4) is computed;
- a data consistency deviation between the first data consistency measure (6a) and the second data consistency measure (6b) is computed;
- the final image (8a, 8b) is reconstructed based on the MRI data (4), wherein, depending on the data consistency deviation, the motion of the object is either compensated based on the motion data (7) in the final image (8a, 8b) or is not compensated in the final image (8a, 8b).

5. Computer-implemented method according to one of claims 3 or 4, wherein the first data consistency measure (6a) depends on $\left‖{E}_{θ} x-y\right‖ ,$ wherein *x* denotes the first preliminary image (5a), *θ* denotes the motion data (7), *E_{θ}* denotes a predefined forward operator, which depends on the motion data (7), and y denotes a part of the MRI data (4), which is used for generating the first preliminary image (5a).

6. Computer-implemented method according to one of claims 1 or 2, wherein
- only a part (4b) of the MRI data (4) for which the motion of the object is neglectable is used for reconstructing the first preliminary image (5a); and/or
- the MRI data (4) comprises a dataset (4b), which corresponds to a preliminary scan of the object, wherein a duration of the preliminary scan is less than ten seconds, and the first preliminary image (5a) is reconstructed based on the dataset (4b).

7. Computer-implemented method according to claim 6, wherein
- the MRI data (4) comprises one or more first datasets (4c) corresponding to one or more first data acquisition shots of the acquisition of the MRI data (4) and one or more second datasets (4d) corresponding to one or more second data acquisition shots of the acquisition of the MRI data (4), wherein the one or more second data acquisition shots are carried out after the one or more first data acquisition shots;
- the first data consistency measure (6a) is computed based on the first preliminary image (5a) with respect to the one or more first datasets (4c) and a second data consistency measure (6b) is computed based on the first preliminary image (5a) with respect to the one or more second datasets (4d); and
- the final image (8a, 8b) is reconstructed based on the MRI data (4), wherein, depending on the first data consistency measure (6a) and the second data consistency measure (6b), the motion of the object is either compensated in the final image (8a, 8b) based on the motion data (7) or is not compensated in the final image (8a, 8b).

8. Computer-implemented method according to claim 7, wherein
- a data consistency variation measure is computed depending on the first data consistency measure (6a) and the second data consistency measure (6b), and the final image (8a, 8b) is reconstructed based on the MRI data (4), wherein, depending on the data consistency variation measure, the motion of the object is either compensated in the final image (8a, 8b) based on the motion data (7) or is not compensated in the final image (8a, 8b); or
- a significance class is determined by applying a machine learning model, MLM, to input data, which depends on or comprises the first data consistency measure (6a) and the second data consistency measure (6b), and the final image (8a, 8b) is reconstructed based on the MRI data (4), wherein, depending on the significance class, the motion of the object is either compensated in the final image (8a, 8b) based on the motion data or is not compensated in the final image (8, 8b).

9. Computer-implemented method according to claim 6, wherein
- for each dataset of a plurality of datasets (4c, 4d) of the MRI data (4), which correspond to a plurality of subsequent data acquisition shots, a respective data consistency measure (6a, 6b) is computed based on the first preliminary image (5a) with respect to the respective dataset (4c, 4d);
- the final image (8a, 8b) is reconstructed based on the MRI data (4), wherein, depending on the data consistency measures (6a, 6b) computed for the plurality of datasets (4c, 4d), the motion of the object is either compensated in the final image (8a, 8b) based on the motion data (7) or is not compensated in the final image (8a, 8b).

10. Computer-implemented method according to claim 9, wherein
- a significance class of the motion of the object is determined by classifying the motion of the object according to at least two significance classes based on the data consistency measures computed for the plurality of datasets; and
- the final image (8a, 8b) is reconstructed based on the MRI data (4), wherein, depending on the determined significance class of the motion of the object, the motion of the object is either compensated in the final image (8a, 8b) based on the motion data (7) or is not compensated in the final image (8a, 8b).

11. Computer-implemented method according to one of the preceding claims, wherein, for reconstructing the first preliminary image (5a) and/or for computing the first data consistency measure (6a), parts of the MRI data (4) are weighted differently depending on respective sampled k-space locations.

12. Computer-implemented method according to one of the preceding claims, wherein the motion data (7) comprises
- one, two or three translation parameters as a function of time during the acquisition of the MRI data (4) and/or one, two, or three rotation parameters as a function of time during the acquisition of the MRI data (4); or
- a motion field as a function of time during the acquisition of the MRI data (4).

13. Data processing system (3), which is configured to carry out a computer-implemented method according to one of the preceding claims.

14. MRI system (1) comprising a data processing system (3) according to claim 13 and an MRI device (2), which is configured to generate the MRI data (4).

15. Computer program product comprising instructions, which when executed by a data processing system (3) cause the data processing system (3) to carry out a computer-implemented method according to one of claims 1 to 12.
